# EUROPEAN PATENT APPLICATION

(11) **EP 4 810 619 A1**
(43) Date of publication of application: **23.09.2026**
(21) Application number: 25165368.9
(22) Date of filing: 21.03.2025
(51) Int. Cl.: C12P 5/02, C12M 1/107, C12M 1/00, C02F 11/04, C05F 17/00, C12M 1/33

(54) **AUTOMATED BIOGAS APPARATUS**

(71) Applicant: Benerg Oy, 00580 Helsinki (FI)
(72) Inventor: Gedik, Burak, 00580 Helsinki (FI); Korkmaz, Canberk, 00580 Helsinki (FI); Surer, Umit, 00580 Helsinki (FI)
(74) Representative: Papula Oy

(57) **Abstract**

The invention relates to an automated apparatus for producing energy from food waste, comprising a feeding unit for feeding the food waste into the apparatus. The apparatus further comprises at least one reaction tank containing anaerobic digesting bacteria for producing biogas from the food waste, a collection system for collecting the produced biogas and an energy production unit. The invention is further directed to a method for producing energy from food waste.

## Description

### FIELD OF THE INVENTION

The invention relates to biogas machines, i.e. machines for producing biogas from waste.

### BACKGROUND OF THE INVENTION

Food waste is a big problem globally. Food waste causes the same amount of emissions as India. Some countries have a functioning process for gathering food waste, which will then be composted in order to produce compost and biogas. This however requires both large facilities for processing the food waste as well as functioning collection of the food waste from both homes, restaurants and shops. In many places this is not implementa-ble, and food waste is simply treated as mixed waste. This is a problem at least if the mixed waste is treated by burning as food waste includes a lot of moisture which harms the burning process.

Another problem with the systems today is that the food waste needs to be transported and centrally treated. This creates a need for transportation, which again causes pollution from trucks used for the transportation. Another problem is the storage of the food waste before it is transported. At least in countries where the temperature rises over 20°C during the day, the food waste will start smelling, as pick up of the waste may not be possible on a daily or even weekly basis.

There is still a need in the art for solutions providing further improvements.

The problems presented above are solved or at least alleviated by the present invention.

### SUMMARY

It is an objective to present an automated apparatus for producing energy from food waste. Another objective is a method for producing energy from food waste.

These and other advantageous objectives are met by a method and apparatus for producing energy from food waste at a building scale without need for transportation of the waste.

According to a first aspect, there is provided a building scale apparatus for producing energy from food waste. The apparatus comprises a feeding unit for feeding the food waste into the apparatus. The apparatus further comprises at least one reaction tank containing anaerobic digesting bacteria for producing biogas from the food waste. The apparatus also comprises a collection system for collecting the produced biogas and an energy production unit.

According to an embodiment, the feeding unit comprises an automated crusher for crushing or grinding the food waste. This involves at least the advantage that the apparatus may function automatically without constant need for monitoring and maintenance.

According to an embodiment, the apparatus comprises at least two reaction tanks. This involves at least the advantage of a faster fermentation process.

According to an embodiment, the apparatus further comprises active carbon filters for filtering the biogas to eliminate H₂S, water vapour and Cox. This involves at least the advantage that advantage that a cleaner biogas is obtained.

According to an embodiment, the energy production unit is a biogas generator for producing electricity. This involves at least the advantage that the energy produced is converted into a form which may be used in the building where the apparatus is installed itself.

According to an embodiment, the energy production unit is a boiler for producing heat. This involves at least the advantage that the energy produced may be used for heating the building wherein the apparatus is installed.

According to an embodiment, the apparatus further comprises a fertilizer collection unit. This involves at least the advantage that the food waste may be utilized fully.

According to a second aspect, there is provided a method for producing energy from food waste. The method comprises feeding food waste into a waste feeding unit of an apparatus for producing electricity from food waste. The method comprises adding water to the crushed food waste and pumping the food waste into a reaction tank, wherein the reaction tank comprises anaerobic digesting bacteria. The method further comprises producing biogas from the food waste in the reaction tank with the anaerobic digesting bacteria and collecting the biogas in a collection tank and converting the biogas into energy.

According to an embodiment, the method comprises a step of crushing or grinding the food waste in order to decrease the particle size and increase the surface area, facilitating the fermentation process with the addition of water. This involves at least the advantage that the fermentation process is enhanced.

According to an embodiment, the method comprises carrying out a further step of filtering the produced biogas out in order to eliminate H₂S, water vapour and COx. This involves at least the advantage that a cleaner biogas is obtained.

According to an embodiment, the method comprises collecting fertilizer resulting from the fermentation process. This involves at least the advantage that no part of the food waste is wasted.

According to an embodiment, the method comprises that the biogas is converted to electricity. This involves at least the advantage that the energy produced is converted into a form which may be used in the building itself.

According to an embodiment, the method comprises that the biogas is converted to heat. This involves at least the advantage that the energy produced may be used for heating the building.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**Figure 1** is a schematic view of an automated biogas apparatus according to one embodiment.
**figure 2** is an illustration of the process of producing biogas according to the same embodiment,
**figure 3** is a top view of a first fermentation tank, according to an embodiment of the invention.
**figure 4** is a top view of a second fermentation tank according to one embodiment of the invention,
**figure 5** illustrates the process of filtration of the biogas,
**figure 6** shows the feeding unit as a front and side view.

### DETAILED DESCRIPTION

In the following description, reference is made to the accompanying drawings, which form part of the disclosure, and in which are shown, by way of illustration, specific aspects in which the present disclosure may be placed. It is understood that other aspects may be utilised, and structural or logical changes may be made without departing from the scope of the present disclosure. The following detailed description, therefore, is not to be taken in a limiting sense, as the scope of the present disclosure is defined be the appended claims.

For instance, it is understood that a disclosure in connection with a described method may also hold true for a corresponding device or system configured to perform the method and vice versa. For example, if a specific method step is described, a corresponding device may include a unit to perform the described method step, even if such unit is not explicitly described or illustrated in the figures. On the other hand, for example, if a specific apparatus is described based on functional units, a corresponding method may include a step performing the described functionality, even if such step is not explicitly described or illustrated in the figures. Further, it is understood that the features of the various example aspects described herein may be combined with each other, unless specifically noted otherwise.

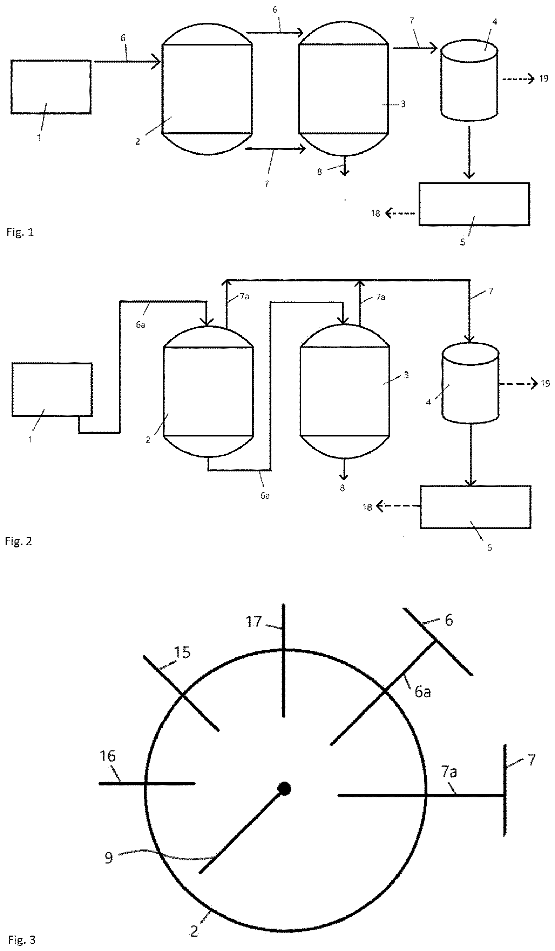

Figure 1 shows a schematic view of an automated biogas apparatus according to one embodiment. The apparatus comprises a waste feeding unit 1, into which food waste is fed. The waste feeding unit 1 comprises a crusher for crushing or grinding the food waste. According to one embodiment the crusher may be an automated crusher. The waste feeding unit may also comprise a water inlet (not shown) for adding water to the crushed food waste to obtain a solution of the crushed food waste. The waste feeding unit preferably has a volume which is large enough to arrange for blending of the crushed food waste with water to obtain a desired density of the blend. This enables controlling pumping and arranging of the blend before being entering the digestion process. To control this, system arranges itself to prepare desired properties by adding water and working rate of crusher in terms of rpm. The apparatus further comprises a feeding pipe 6 for feeding the solution of food waste and water into a first fermentation tank 2 from the waste feeding unit 1. According to one embodiment multiple fermentation tanks may be used. In this case a feed pipe 6 is used to connect the first fermentation tank 2 to the second fermentation tank 3 and so on. The use of more than one digester has the advantage of easier separation of fully fermented food waste as new waste will be added by the users every day, which will be mixed with the food waste already in the fermentation tank. The fermentation tanks mey be mixed with motor-controlled system to enable gas to float to the surface. Having more than one digester enables for easier to control of the process.

Fermentation tanks 2 and 3 comprise anaerobic bacteria to enhance fermentation of the food waste and produce biogas. Fermentation tanks 2 and 3 are connected to a biogas control tank 4 by means of bio gas lines 7 for transporting the produced biogas from fermentation tanks 2 and 3 to control tank 4. Control tank 4 may be connected to a generator 5 for converting biogas into electricity. The produced electricity is then collected at electricity collection point 18. The biogas in the control tank 4 may also be transferred further by a biogas production line 19 i.e. to be used for heating purposes.

Figure 2 shows an example of the steps of the process of producing biogas with the apparatus according to the invention. The food waste is fed into a waste feeding unit 1. The waste feeding unit comprises a crusher for crushing or grinding the food waste. According to one embodiment the food waste may be crushed with automated crushers to decrease particle size, in order to flourish the anaerobic digesting process. At this point water is added to the food waste in order to form a liquid. The density of the liquid containing the crushed food waste is around 1.1 kg/m³. The liquid is then transferred 6a to a first fermentation tank 2 from the waste feeding unit 1. If multiple fermentation tanks are used, the liquid is further transported 6a to a second fermentation tank 3.

Fermentation tanks 2 and 3 comprise anaerobic bacteria to enhance fermentation of the food waste and produce biogas. The biogas produced in fermentation tanks 2 and 3 is then transferred 7a to a biogas control tank (4).

From control tank 4 the biogas may be transferred to a generator 5 for converting said biogas into electricity. The produced electricity may then be collected at electricity collection point 18. The biogas in the control tank 4 may also be transferred further by a biogas production line 19 i.e. to be used for heating purposes.

Figure 3 presents a top view of a first fermentation tank 2, according to an embodiment of the invention. The fermentation tank comprises a mixer 9 for a mixing the solution of food waste and water to form a homogeneous mixture in the fermentation tank 2. Here 6, 6a represents the waste line for transferring the crushed food waste to the fermentation tank. 7, 7a represents the biogas line for transferring the produced biogas out of the fermentation tank 2.

The fermentation tank 2 may further be equipped with a pressure control sensor 15, a distance control sensor 16, and a heat control 17 sensor for controlling the pressure, volume, and temperature values in the fermentation tank 2, in order to create an optimal environment for the fermentation process.

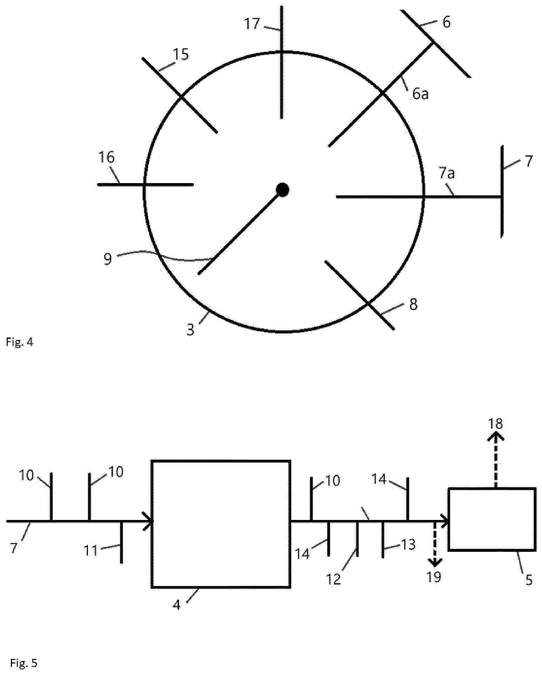

Figure 4 shows a top view of a second fermentation tank 3 according to an embodiment. The second fermentation tank may further comprise a fertilizer collection tank for collection of fertilizer which is produced as a biproduct in the process.

Figure 5 shows the process of filtration of the biogas. The produced biogas is filtrated to achieve a cleaner end product. When the produced biogas is transferred from the fermentation tank it is lead through a filtering stage. This stage may comprise filtering of the biogas through multiple filters.

According to one embodiment the produced biogas goes through a carbon filter 10, a water/biogas filter 11, and a biogas filter 13 process. According to the embodiment illustrated in figure 5, the biogas is first filtered through two carbon filters and one water/biogas filter to eliminate H₂S, water vapour and Cox. Then, the biogas enters a control tank before again being filtered through a carbon filter 10. After this the biogas passes through a safety valve 14, and a biogas regulator 12, a further biogas filter 13 and a safety valve 14 and reaches a ready state before use.

The filtered biogas may be used in two ways, as a biogas 19 or by converting the biogas into electricity 18 with a generator 5. 19 is shown as biogas used for heating etc. and 18 is shown as biogas used for electricity generation in figure 5.

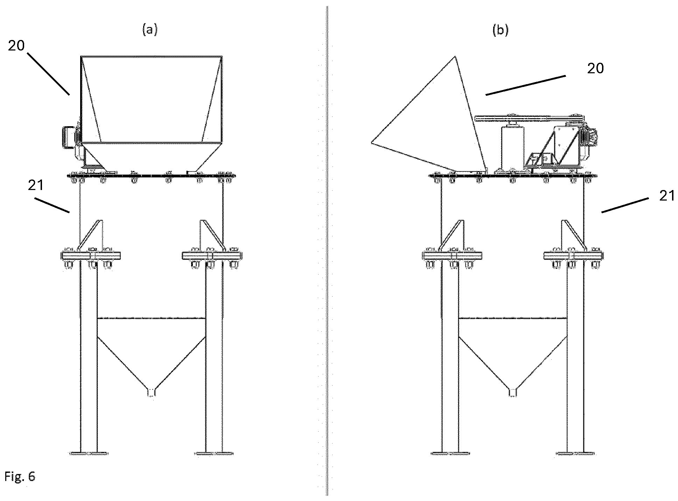

Figure 6 shows a feeding unit according to an embodiment both as a front view (a) and as a side view (b). The feeding unit comprises a part for receiving the food waste 20, a housing 21 comprising an automated crusher for crushing or grinding the food waste and pumps (not shown) for adding water to the food waste and for pumping the resulting solution into a first fermentation tank.

According to an embodiment the automated apparatus for producing biogas is a small-scale biogas apparatus. With small scale here is meant an apparatus which is suitable for handling waste food to an amount of 0,5-120 kg daily. According to one embodiment the outer dimensions of the apparatus are scaled according to the amount of food waste the apparatus can handle. Some examples of different scales of the apparatus can be seen in the below table:

| Daily capacity (kg) | kWh | Length of apparatus (mm) | Width of apparatus (mm) | Height of apparatus (mm) |
|---|---|---|---|---|
| 60 | 45-60 | 5900 | 3350 | 2900 |
| 80 | 65-80 | 6200 | 3800 | 3150 |
| 100 | 75-100 | 6300 | 3900 | 3300 |
| 120 | 90-120 | 6750 | 4200 | 3350 |

It is obvious to a person skilled in the art that with the advancement of technology, the basic idea of the invention may be implemented in various ways. The invention and its embodiments are thus not limited to the examples described above, instead they may vary within the scope of the claims.

## Claims

1. An automated apparatus for producing energy from food waste, comprising:
a feeding unit for feeding the food waste into the apparatus;
at least one reaction tank containing anaerobic digesting bacteria for producing biogas from the food waste;
a collection system for collecting the produced biogas; and
an energy production unit.

2. The apparatus of claim 1, wherein the waste feeding unit comprises an automated crusher for crushing or grinding the food waste.

3. The apparatus of claim 1 or 2, wherein the apparatus comprises at least two reaction tanks.

4. The apparatus of any of the preceding claims wherein the apparatus further comprises active carbon filters for filtering the biogas to eliminate H₂S, water vapour and COx.

5. The apparatus of any of the preceding claims wherein the energy production unit is a biogas generator for producing electricity.

6. The apparatus of any of the preceding claims wherein the energy production unit is a boiler for producing heat.

7. The apparatus of claim 1 or 2 wherein the apparatus further comprises a fertilizer collection unit.

8. A method for producing energy from food waste comprising the steps of:
feeding food waste into a waste feeding unit of an apparatus for producing electricity from food waste;
adding water to the crushed food waste;
pumping the food waste into a reaction tank;
Wherein the reaction tank comprises anaerobic digesting bacteria;
producing biogas from the food waste in the reaction tank with the anaerobic digesting bacteria;
collecting the biogas in a collection tank;
converting the biogas into energy.

9. The method of claim 8, further comprising a step of crushing or grinding the food waste in order to decrease the particle size and increase the surface area, facilitating the fermentation process with the addition of water.

10. The method of claim 8 or 9, comprising a further step of filtering the produced biogas is carried out in order to eliminate H₂S, water vapour and COx.

11. The method of any of the preceding claims further comprising a step of collecting fertilizer resulting from the fermentation process.

12. The method of any of claims 8-11 wherein the biogas is converted to electricity.

13. The method of any of claims 8-11 wherein the biogas is converted to heat.
